# EUROPEAN PATENT APPLICATION

(11) **EP 4 407 020 A1**
(43) Date of publication of application: **31.07.2024**
(21) Application number: 23153442.1
(22) Date of filing: 26.01.2023
(51) Int. Cl.: C12M 1/00

(54) **CHAMBER SYSTEM FOR CELL-FREE PRODUCTION OF PROTEINS OF INTEREST**

(71) Applicant: Technische Universität München, 80333 München (DE)
(72) Inventor: VOGELE, Kilian, 82399 Raisting (DE); SIMMEL, Friedrich, 80336 München (DE); WINZIG, Franziska, 65510 Idstein (DE)
(74) Representative: Weickmann & Weickmann PartmbB

(57) **Abstract**

The invention relates to a chamber system for cell-free production of proteins of interest, comprising:
at least one reaction chamber (10), being configured to hold a reaction solution (12) and being configured to produce proteins of interest using the reaction solution (12);
at least one feeding chamber (20), being disposed adjacent to the at least one reaction chamber (10) and being configured to hold a feeding solution (22);
at least one semi-permeable membrane (30), connecting the at least one feeding chamber (20) with the at least one reaction chamber (10), wherein the semi-permeable membrane (30) is configured to allow diffusing at least part of the feeding solution (22) from the at least one feeding chamber (20) into the at least one reaction chamber (10);
wherein the at least one feeding chamber (20) comprises a feeding chamber access (21) that is configured to allow access to the feeding solution (22) in the at least one feeding chamber (20).

## Description

### Field of the invention

The present disclosure lies in the field of synthetic biology and relates to a chamber system for cell-free production of proteins of interest.

### Technical background

Cell-free transcription and translation is an *in vitro* synthetic biology tool. It consists of all cellular components necessary for expression such as the ribosome, tRNA synthetases and transcription factors. With the addition of a DNA template, it can express the encoded proteins. Primarily, there are two ways to obtain the necessary protein-based components. In the commercial PURE system, all necessary proteins are recombinantly produced and individually isolated. Second, isolation of a crude cell extract from bacteria, usually *E. coli,* is possible. In this preparation method, however, components are also present in the cell extract that are not primarily required for expression, such as DNases and RNases. However, other starting cells can also be used for the preparation, such as standardized rat liver cells, human and rabbit reticulocytes, ascites cells, and wheat germ. To prepare such a cell extract, the cells are first lysed and then the genome and membrane components are removed. In addition, a dialysis step is usually also performed to remove smaller cell components such as metabolites. These small metabolites, such as amino acids and nucleoside triphosphates, are then added back in a controlled manner. Usually, the cell-free system also contains an ATP- regeneration system with which it is possible to get a higher yield of target protein. For cell-free systems made of purified proteins only, the protein yield is around 200 pg/ml, for crude cell extracts it can be up to 2 mg/ml. The advantage of the cell extract system is its open nature, which makes it possible to stimulate protein expression simply by addition of DNA. This opens up broad possibilities for the application of such cell-free systems. These range from prototyping of biological networks, to artificial cell systems to metabolic and chemical design of reaction cascades to the production of bacteriophages. Adding the genome of a phage to the cell extract results in the expression of phage proteins and the self-sustained assembly of full-scale bacteriophages. Here, the yield can be improved by adding molecules such as polyethylene glycol. These molecules bind water and create a more concentrated environment. This is necessary because there is only 25 to 30 times less protein concentration in the cell extract than in the cytosol of a bacterial cell. With cell-free expression of bacteriophages, all things considered, it is possible to obtain titers of up to 10¹² active phages per millilitre in about 6 hours. This has been successfully demonstrated in experiments for RNA phages such as MS2, circular DNA phages such as phi 174, as well as linear DNA phages such as T7 and T4. The more proteins are encoded on a phage's genome, the lower the resulting phage concentration. However, even for large phages, such as the T4 with more than 280 coding genes, the resulting phage concentration is still 10⁹ active phages per millilitre, which is well above the medically relevant titer of 10⁶ active phages per millilitre. The advantage of this technique is the defined composition of the cell-free system, which eliminates hazards from contaminants such as prophages. Thus, the *in vitro* production of therapeutic bacteriophages provides a promising alternative to conventional phage production in the pathogenic host bacterium.

Protein synthesis in cell-free systems quickly reaches its limits when it comes to achieving particularly high yields. The reason for this is that additional factors such as ribonucleoside tri-phosphates (rNTPs), but especially ATP and GTP, are consumed. As a result, they are then no longer available for further synthesis. In addition, the pH changes by processes like turning over NAD⁺, which inhibits further reaction steps. Other limiting factors include degradation of mRNA and accumulation of by-products at inhibitory concentrations. In addition, the yield is negatively correlated with the 'metabolic load' of the genome. Because of these inhibitory factors, chambers have been developed that continuously supply the cell-free system with buffer consisting of the limiting factors via a dialysis membrane or flow chamber. Through these membranes or flow-through chambers, buffer exchange occurs due to the constant influx of metabolites, energy carrying molecules and cofactors into the buffer, and at the same time, the synthesized proteins cannot diffuse outward. This ensures a constant supply of the required small molecules as well as a stable pH over the reaction time, leading to a longer course of the reaction and a higher protein yield.

However, there is still a need for a chamber system providing an improved cell-free production of proteins of interest using buffers to support the protein production.

### Summary

The present invention is defined by the subject-matter of the independent claims. Additional features of the invention are presented in the dependent claims.

According to an aspect of the invention, a chamber system for cell-free production of proteins of interest comprises the following: At least one reaction chamber, being configured to hold a reaction solution and being configured to produce proteins of interest using the reaction solution, at least one feeding chamber, being disposed adjacent to the at least one reaction chamber and being configured to hold a feeding solution and at least one semi-permeable membrane, connecting the at least one feeding chamber with the at least one reaction chamber. The semi-permeable membrane is configured to allow diffusing at least part of the feeding solution from the at least one feeding chamber into the at least one reaction chamber. The at least one feeding chamber comprises a feeding chamber access that is configured to allow access to the feeding solution in the at least one feeding chamber.

The term "feeding solution", as used herein, is also referred to as buffer solution. Typically, the "feeding solution" is different from the reaction solution. The feeding solution comprises components for supporting the protein production. The feeding solution may comprise one or more of the compounds selected from the group consisting of amino acids, HEPES, ATP, GTP, CTP, UTP, tRNA, coenzyme A, NAD, cAMP, folinic acid, spermidine, PEP or 3-PGA, PEG-8000, d-Ribose, Maltodextrin, Glucose, magnesium-glutamate and potassium-glutamate. The feeding solution preferably supports, besides the protein expression and self-assembly an energetically-driven packaging of DNA in phages of the reaction solution, which in many cases is an ATP-dependent process, but which can only take place after protein expression and self-assembly of the phage structure in the reaction solution.

The term "reaction solution", as used herein comprises at least one template encoding at least one protein to be produced and, for example, a bacterial cell extract, such as a cell lysate. The reaction solution contains the cellular machinery (e.g. replication, transcription and translation machinery, such as ribosomes, tRNA synthetases and if needed transcription factors) necessary to facilitate production of the protein and/or bacteriophage. The template can be, but is not limited to a nucleic acid sequence e.g. a PCR product, native DNA/RNA, chemically synthesized templates, plasmids, or a yeast artificial chromosome.

"Cell lysate" as used herein refers to a composition comprising the components of cells of a microorganism, in particular a bacterium, after lysis. The cell lysate is therefore void of intact cells, i.e. cell-free. Typically, the cell lysate is free of host DNA. Preferably, the cell lysate is free of host DNA and membranes. Moreover, the cell lysate may be free of small metabolites. The cell lysate may comprise the transcription and translation machinery of the organism.

The term "protein of interest", as used herein, as used herein, refers to a protein, which is encoded on the Template, which externally added to the system.. The term includes polypeptides. It may comprise natural and non-natural amino acids. Preferably, it comprises natural amino acids. Preferably, the "protein of interest" is a viral protein, more preferably, the viral protein is a bacteriophage protein. In one embodiment, the protein is a recombinant protein, such as a recombinant viral protein, e.g. a recombinant bacteriophage protein. Preferably, the protein of interests comprises synthetic proteins.

In a specific embodiment, the protein of interest is produced in high yields.

In one embodiment, the chamber system is for cell-free multi-step protein synthesis, such as bacteriophage production.

A bacteriophage, also termed phage, is a virus that infects the microorganisms, namely bacteria or archaea. It is composed of capsid proteins that encapsulate a DNA or RNA genome. After infection of their genome into the cytoplasm, bacteriophages replicate in the microorganism using the transcription and translation apparatus of the microorganism. Phages are classified by the international Committee on Taxonomy of Viruses according to morphology and nucleic acid, including *Ackermannviridae, Myoviridae, Siphoviridae, Podoviridae, Lipothrixviridae, Rudiviridae, Ampullaviridae, Bicaudaviridae, Clavaviridae, Corticoviridae, Cystoviridae, Fuselloviridae, Globuloviridae, Inoviridae, Leviviridae, Microviridae, Plasmaviridae, Pleolipoviridae, Portogloboviridae, Spharolipoviridae, Spiraviridae, Tectiviridae, Tristromaviridae, Turriviridae.*

Preferably, the semi-permeable membrane comprises a dialysis membrane.

The term "semi-permeable membrane", as used herein, refers to a type of biological or synthetic, polymeric membrane that will allow certain molecules or ions to pass through it by osmosis. Preferably, the semi-permeable membrane comprises a dialysis membrane. In one embodiment, the semi-permeable membrane has a pore size of 0.1-10 nm. Preferably, the semi-permeable membrane has a pore size of 1-5 nm. Preferably, the semi-permeable membrane has a pore size of about 2 nm.

Preferably, the at least one semi-permeable membrane is made of a hydrophilic material.

Preferably, the at least one semi-permeable membrane is a cellulose membrane.

In other words, the feeding solution can be supplemented during the reaction if necessary, for example if a sequential reaction in the cell extract is involved.

Preferably, the chamber system is manufactured using a filament printer, in particular using polylactic acid, PLA.

Preferably, one of the at least one feeding chambers is disposed adjacent to a plurality of reaction chambers.

Preferably, a plurality of feeding chambers are disposed adjacent to the at least one reaction chamber.

Preferably, the reaction chamber incubates the reaction solution to produce, or in other words assemble, the proteins.

Preferably, the chamber system comprises a first feeding chamber and a second feeding chamber, wherein the first feeding chamber and the second feeding chamber are disposed adjacent to the at least one reaction chamber at opposing sides of the at least one reaction chamber.

Preferably, the reaction chamber is rinsed beforehand to increase the stability of the reaction in the reaction chamber. For this purpose, the chamber is rinsed with a Phosphate buffered saline, PBS, (salt buffer) and/or a Bovine serum albumin, BSA, (protein solution). Thus, the reaction works better with rinsing and in particular works best with BSA. In other words, a surface of the reaction chamber is coated with PBS and/or BSA. Consequently, rinsing the reaction chamber before filling the reaction chamber with the reaction solution provides higher protein yield.

In a preferred embodiment, the feeding chamber access is disposed on a top surface of the at least one feeding chamber.

In a preferred embodiment, the feeding chamber access is configured to allow access to the feedings solution during production of the proteins of interest.

The feeding chamber access to the feeding chamber allows to continuously replenish the feeding solution and/or adjust a feeding solution composition.

The chamber system allows that the composition of the cell-free reaction can be changed from the outside during the reaction without diluting the proteins. For this purpose, the cell-free reaction is carried out in a reaction chamber which is connected to a feeding chamber by a suitable semi-permeable membrane. Thus, by this imbalance of the reaction solution and the feeding solution, a better yield of the reaction can be obtained. Due to the direct access to the feeding chamber, the feeding solution can dynamically be replenished and/or adjusted, further improving the yield of the reaction in the reaction chamber. In particular, at different stages of the reaction in the reaction chamber, different feeding solutions can be used in the feeding chamber. In addition, the duration of the reaction itself can be further extended, allowing for a longer production of proteins of interest.

In a preferred embodiment, the at least one semi-permeable membrane is vertically oriented between the at least one reaction chamber and the at least one feeding chamber.

The vertical feeding of the feeding solution reduces problems due to sedimentation in the feeding solution.

The phrase "problems due to sedimentation", as used herein, refers to an accumulation of molecular aggregates, cellular debris or other by-products on the semi-permeable membrane. This accumulation leads to an every-increasing inhibition of solutes or solution passing the membrane, which in turn impedes the long-term upkeep of cell-free protein production, in particular high yield protein production and/or multi-step protein production, such as bacteriophage production.

In a preferred embodiment, the at least one semi-permeable membrane is fixed between the at least one reaction chamber and the at least one feeding chamber using an adhesive.

Adhering the semi-permeable membrane to the feeding chamber and/or the reaction chamber provides an improved tightness between the semi-permeable membrane and the respective chambers. Consequently, leakage out of the feeding chamber and/or the reaction chamber is prevented. Furthermore, undesirable mixing of feeding solution between adjacent feeding chambers is prevented. Furthermore, undesirable mixing of reaction solution between adjacent reaction chambers is prevented.

Preferably, the adhesive comprises silicon compounds.

Silicone compounds are relatively easy to use and allow a high tightness between the semi-permeable membrane, the reaction chamber and the feeding chamber.

Preferably, the adhesive is at least in part biocompatible. Thus, the reaction in the reaction chamber is not disturbed by the adhesive.

Preferably, the adhesive comprises Polydimethylsiloxane. Further preferably, the adhesive is Picodent Twinsil.

In a preferred embodiment, the protein of interest is a bacteriophage protein.

In a preferred embodiment, the chamber system comprises at least one reaction comb, wherein the at least one reaction comb comprises the at least one reaction chamber, and at least one feeding comb, wherein the at least one feeding comb comprises the at least one feeding chamber. The at least one reaction comb is disposed adjacent to the at least one feeding comb in a width direction. Between the at least one reaction comb and the at least one feeding comb the at least one semi-permeable membrane is disposed, connecting the at least one reaction chamber with the at least one feeding chamber.

The comb structure, having a bigger length than width, allows improved further processing of the reaction solution, in particular by a plate reader. For example, the plate reader is a CLARIOstar of BMG LABTECH.

In a preferred embodiment, the reaction comb comprises a plurality of reaction chambers that are disposed adjacent to each other along a length direction; perpendicular to the width direction.

In a preferred embodiment, the feeding comb comprises a plurality of feeding chambers that are disposed adjacent to each other along the length direction perpendicular to the width direction.

In a preferred embodiment, between the at least one reaction comb and the at least one feeding comb only one semi-permeable membrane is disposed, connecting the at least one reaction chamber with the at least one feeding chamber.

In a preferred embodiment, the chamber system comprises a first feeding comb and a second feeding comb, wherein the first feeding comb and the second feeding comb are disposed adjacent to the at least one reaction comb at opposing sides of the at least one reaction comb in the width direction.

The first feeding comb and the second feeding comb are structurally the same.

Using a feeding comb from each side of the reaction chamber increases the contact area between the reaction chamber and the feeding chambers. Thus, diffusion of the feeding solution into the reaction solution can be increased.

In a preferred embodiment, the first feeding comb and the second feeding comb comprise fastening means that extend towards each other in the width direction. The at least one reaction comb comprises at least one fastening opening through which the fastening means of the first feeding comb and the second feeding comb extend. The fastening means are configured to fasten the first feeding comb and the second feeding comb together, holding the reaction comb between the first feeding comb and the second feeding comb.

The fastening means preferably comprise at least one protrusion and at least one recess. The protrusion of the first feeding comb extends through the fastening opening of the reaction comb into the recess of the second feeding comb. The protrusion of the second feeding comb extends through the fastening opening of the reaction comb into the recess of the first feeding comb. The protrusion and the recess of the first feeding comb and the second feeding comb are adjacent to each other, respectively. Thus, the fastening means extend through fastening opening of the reaction comb adjacently. Consequently, the fastening opening of the reaction comb is at least of the size of two times the cross-section area of the protrusion.

This allows to align the first feeding comb and the second feeding comb to the reaction comb, when adhering the semi-permeable membrane to the first feeding comb, the second feeding comb and/or the reaction comb.

This allows for an improved fastening of the first feeding comb and the second feeding comb with the reaction comb.

In a preferred embodiment, the system comprises a box, wherein the box comprises a bottom part, configured to receive the at least one reaction chamber and the at least one feeding chamber, and a cover part, configured to cover the bottom part.

In a preferred embodiment, the semi-permeable membrane has a pore size of less than or equal to 2 nm.

According to an aspect of the invention, a method for cell-free production of proteins of interest using a chamber system, as used herein, comprises the steps. Providing the reaction solution to the at least one reaction chamber and the feeding solution to the at least one feeding chamber. Incubating the reaction solution, thereby producing the proteins of interest.

Preferably, the chamber system, as used herein, is used for cell-free production of proteins of interest.

The subject matter of the invention will be explained in more detail in the following text with reference to preferred exemplary embodiments, which are illustrated in the attached drawings, in which:

### Brief description of the drawings

Exemplary embodiments of the invention will be described in the following with reference to the accompanying drawings:
Fig. 1 shows a schematic view of a chamber system for cell-free production of proteins of interest in accordance with a first embodiment;
Fig. 2 shows a schematic view of a chamber system for cell-free production of proteins of interest in accordance with a second embodiment;
Fig. 3 shows a schematic view of a chamber system for cell-free production of proteins of interest in accordance with a third embodiment;
Fig. 4 shows a schematic view of a reaction comb;
Fig. 5 shows a schematic view of a feeding comb;
Fig. 6 shows a schematic view of a bottom part of a box;
Fig. 7 shows a diagram comparing protein production with different rinsing of the reaction chamber;
Fig. 8a shows a diagram comparing protein production with and without feeding solution in a chamber system with horizontal semi-permeable membrane;
Fig. 8b shows a schematic chamber system with horizontal semi-permeable membrane;
Fig. 9 shows a diagram comparing different relative levels of buffer solution and reaction solution;
Fig. 10 shows a diagram comparing protein production with and without feeding solution in a chamber system with vertical semi-permeable membrane; and
Fig. 11 shows a diagram comparing protein production with different conditions.

The reference symbols used in the drawings, and their meanings, are listed in summary form in the list of reference symbols. In principle, identical assembly parts are provided with the same reference symbols in the figures.

### Detailed description of exemplary embodiments

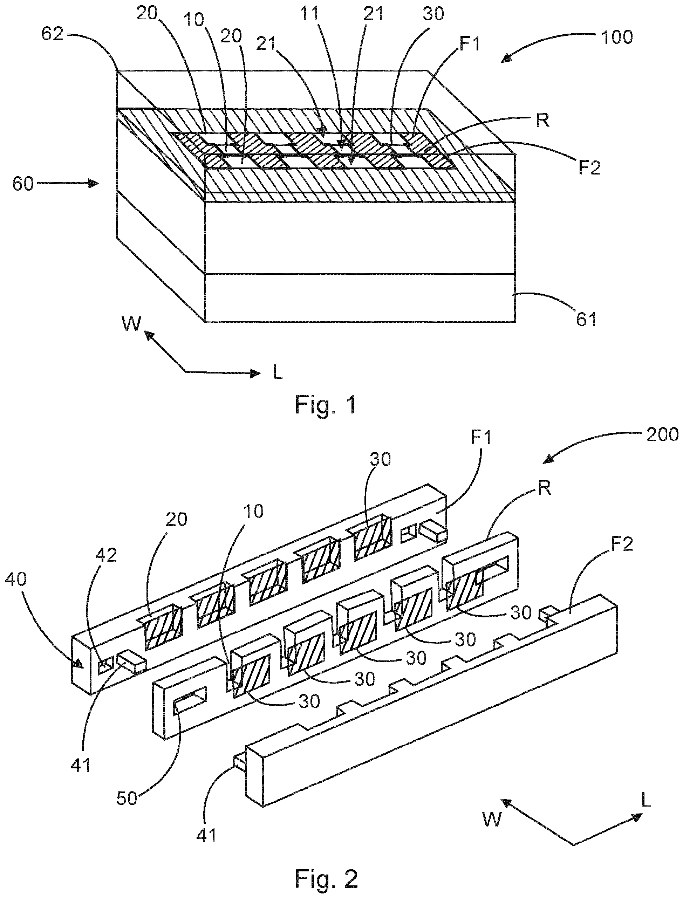

Fig.1 shows a schematic view of a chamber system 100 for cell-free production of proteins of interest in accordance with a first embodiment. The chamber system 100 comprises a box 60 that consist of a bottom part 61 and a top part 62.

In the bottom part 61 of the box 60, a reaction comb R is disposed between a first feeding comb F1 and a second feeding comb F2. The reaction comb R comprises a plurality of reaction cambers 10, in this case four reaction chambers 10. The plurality of reaction chambers 10 are disposed adjacent to each other in a length direction L along the reaction comb R. The first feeding comb F 1 and the second feeding comb F2 each comprise a plurality of feeding chambers 20, in this case four feeding chambers 20. The plurality of feeding chambers 20 are disposed adjacent to each other in a length direction L along the respective feeding comb F1, F2.

The reaction comb R, the first feeding comb F 1 and the second feeding comb F2 are disposed in such a way that each reaction chamber 10 borders on one of the feeding chambers of the first feeding comb F 1 in the width direction W and one of the feeding chambers of the second feeding comb F2 against the width direction W. Thus, each reaction chamber 10 borders on two feeding chambers 20, in particular on opposite sides of the reaction chamber 10. The reaction chamber 10 and the feeding chambers 20 are separated by a semi-permeable membrane 30. Thus a semi-permeable membrane 30 is located between the first feeding comb F1 and the reaction comb R and another semi-permeable membrane 30 is located between the reaction comb R and the second feeding comb F2.

The reaction chambers 10 comprise a reaction solution that is used to produce proteins of interest in the reaction chamber 10. The feeding chambers 20 comprise a feeding solution. The feeding solution comprises components for supporting the protein production. The first semi-permeable membranes 30 and the second semi-permeable membrane 30 are configured to allow diffusing at least part of the feeding solution from the feeding chambers 20 into the bordering reaction chamber 10.

The first step involves the preparation of an E.coli cell lysate. Here, an E.coli based cell extract is used as they have the highest expression capacity so far and its composition is well known. The second step involves the preparation of the genetic information (DNA/RNA), for *in vitro* expression. This genetic information must be isolated, or physically available in the form of a PCR product, native DNA/RNA, chemically synthesized, as a plasmid, or as yeast artificial chromosomes (list not exhaustive). The third step is to prepare a chamber. In this case, a filament printer was used to print the design shown in Figure 1 using PLA.

Fig. 2 shows a schematic view of a chamber system 200 for cell-free production of proteins of interest in accordance with a second embodiment. In this embodiment, the reaction comb comprises five reaction chambers 10. The first reaction comb F1 and the second reaction comb F2 each comprise five feeding chambers 20. The explosive view on the chamber system indicates that each connection between reaction chamber 10 and feeding chamber 20 is separated by a semi-permeable membrane 30.

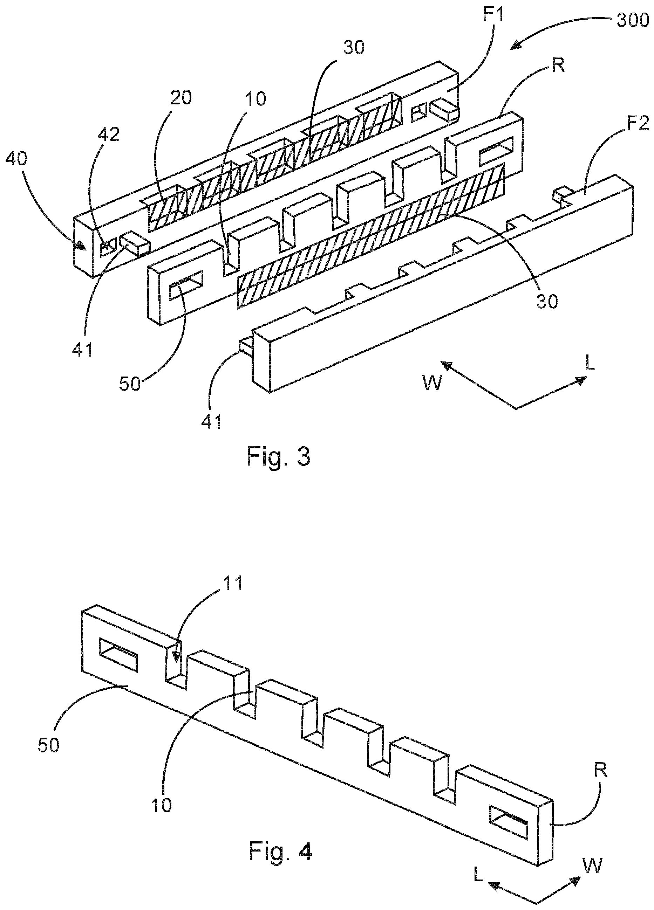

Fig. 3 shows a schematic view of a chamber system 300 for cell-free production of proteins of interest in accordance with a third embodiment. Compared to the chamber system in accordance with the second embodiment of Fig. 2, only two semi-permeable membranes are used to separate the feeding chambers 20 of the first feeding comb F 1 and the second feeding comb F2 from the reaction chamber 10 reaction comb R. In other words, one single semi-permeable membrane 30 extends in length direction L to separate all feeding chambers 20 of the first feeding comb F 1 from the reaction chambers 10 of the reaction comb R and one single semi-permeable membrane 30 extends in length direction L to separate all feeding chambers 20 of the second feeding comb F2 from the reaction chambers 10 of the reaction comb R.

Fig. 4 shows a schematic view of a reaction comb R. The reaction comb R has a general cuboid shape, which has a length in length direction L that is higher than a width in width direction W, wherein the length direction L is perpendicular to the width direction W. The reaction comb R comprises a plurality of reaction chambers 10, in this case four reaction chambers 10. The reaction chambers 10 are formed by recesses that are introduced into the cuboid shape from a top side of the reaction comb R. The recesses forming each reaction chamber 10 extend along the complete width of the respective reaction comb R. In other words, the reaction chambers 10 are open from the top side and on both sides in the width direction W. In an assembled states, as for example seen in fig. 1, the open sides in width direction W of the reaction chambers 10 are closed by the semi-permeable membrane 30 and go over to an adjacent feeding chamber 20 of an adjacent feeding comb F.

The open side of the reaction chambers 10 on the top side of the reaction chambers 10 provide a reaction chamber access 11, which allows to access the reaction solution that is disposed in the reaction chambers 10.

Consequently, the four recesses forming the reaction chambers 10 lead to five finger portions extending in a height direction H, wherein the height direction H is perpendicular to the width direction W and the length direction L. In other words, along the length direction L of the reaction comb R, the finger portions and the recesses, in other words the reaction chambers 10, alternate. The finger portions, in particular the two finger portions at the respective ends in the length direction L of the reaction comb R comprise a fastening opening 50 in width direction W through which the fastening protrusion 51 of the adjacent feeding combs can extend.

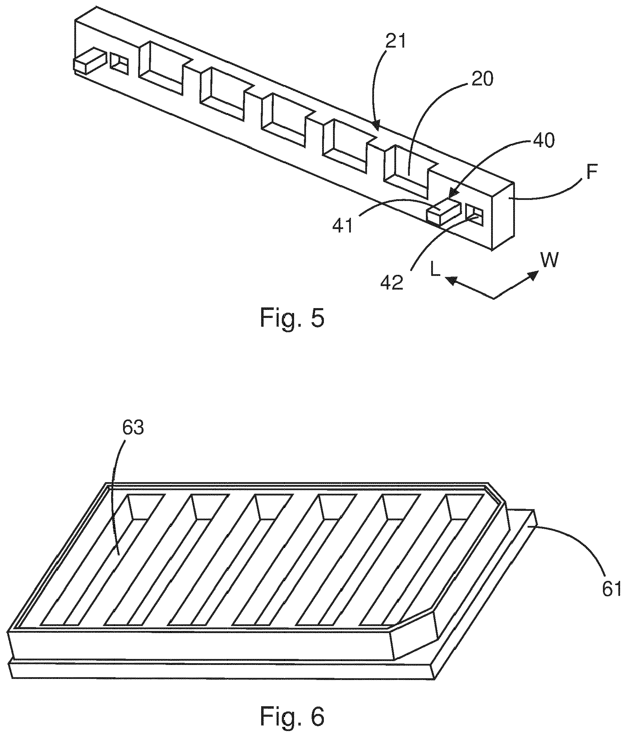

Fig. 5 shows a schematic view of a feeding comb F. The feeding comb F has a general cuboid shape, which has a length in length direction L that is higher than a width in width direction W, wherein the length direction L is perpendicular to the width direction W. The feeding comb F comprises a plurality of reaction chambers 20, in this case four feeding chambers 20. The feeding chambers 20 are formed by recesses that are introduced into the cuboid shape from a top side of the feeding comb F. The recesses forming each feeding chamber 20 extend from one end of the feeding comb F in the width direction W without reaching to the other end of the feeding comb F in the width direction W. The side of the feeding comb F, the feeding chamber is open is referred to as connecting side. In other words, the feeding chambers 20 are open at the top side and on one side in the width direction W. In an assembled states, as for example seen in fig. 1, the open side in width direction W of the reaction chambers 10 is closed by the semi-permeable membrane 30 and goes over to an adjacent reaction chamber 10 of an adjacent reaction comb R.

The open side of the feeding chambers 20 on the top side of the feeding chambers 20 provide a feeding chamber access 21, which allows to access the feeding solution that is disposed in the feeding chambers 20.

Consequently, the four recesses forming the feeding chambers 20 lead to five finger portions extending in a height direction H, wherein the height direction H is perpendicular to the width direction W and the length direction L. In other words, along the length direction L of the feeding comb F, the finger portions and the recesses, in other words the feeding chambers 10, alternate. The finger portions, in particular the two finger portions at the respective ends in the length direction L of the feeding comb R comprise fastening means 40, comprising a fastening recess 41 and a fastening protrusion 41, which is disposed adjacent to the fastening recess 41 in length direction L. The fastening protrusion 41 extends from the finger portion against the width direction W. Thus, the fastening protrusion extends form the feeding comb F against the width direction from the connecting side.

Due to the structure of the feeding comb F, the first feeding comb F 1 and the second feeding comb F2 of fig. 2 may be the same.

Fig. 6 shows a schematic view of a bottom part 61 of a box. The bottom part 61 comprises a plurality of comb recesses 63, which are configured to receive a reaction comb R, sandwiched between a first feeding comb F 1 and a second feeding comb F2. The amount of comb recesses 63 preferably depends on the capabilities of a plate reader that is used to analyze the reaction solution in the reaction comb R. In other words, either the plate reader is able to receive a full reaction comb R - feeding comb F combination or the whole bottom part 61 of the box.

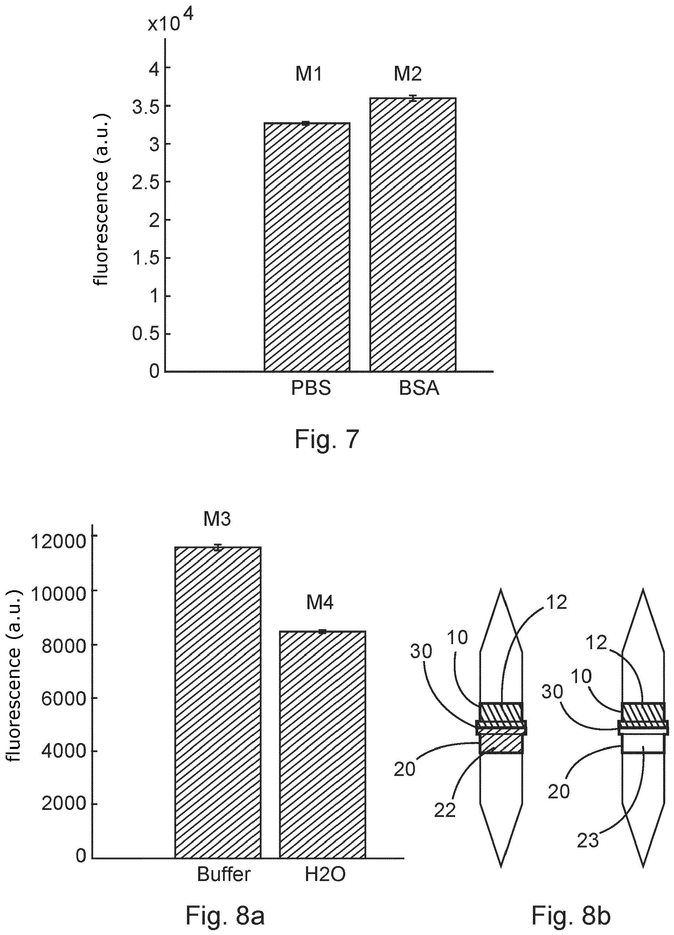

Fig. 7 shows a diagram comparing protein production with different rinsing of the reaction chamber. The diagram shows an endlevel fluorescence of the produced protein in arbitrary units, a.u., with different rinsing of the reaction chamber before filling the reaction solution into the reaction chamber. In other words, the reaction chamber is rinsed beforehand to increase the stability of the reaction in the reaction chamber. For this purpose, the chamber is rinsed with a Phosphate buffered saline, PBS, (salt buffer) and/or a Bovine serum albumin, BSA, (protein solution). A first measurement M1 indicates that protein production in a chamber system after rinsing the reaction chamber with PBS reaches a fluorescence of about 33000 a.u. A second measurement M2 indicates that protein production in a chamber system after rinsing the reaction chamber with BSA reaches a fluorescence of about 36000 a.u. Consequently, rinsing the reaction chamber with BSA offers a higher yield of protein production compared to rinsing the reaction chamber with PBS.

Fig. 8a shows a diagram comparing protein production with and without feeding solution in a chamber system with a horizontal semi-permeable membrane. The diagram indicates an endlevel fluorescence measurement of a horizontal transcription-translation, TX-TL, reaction with mTurquoise (mTurq) as a fluorescent protein.

A third measurement M3 indicates that protein production in a chamber system with a horizontal semi-permeable membrane and a feeding solution 22 reaches a fluorescence of about 12000 a.u. A fourth measurement M4 indicates that protein production in a chamber system with a horizontal semi-permeable membrane and no feeding solution 22, but just H2O 23 that does not affect the reaction in the reaction chamber reaches a fluorescence of about 8000 a.u. Consequently, using a feeding solution 22 to support the protein production improves the yield of the protein production.

Fig. 8b shows a schematic chamber system with horizontal semi-permeable membrane 30. On the left side, the system comprises a reaction chamber 10 holding a reaction solution 12 and a feeding chamber 20 holding a feeding solution 22. The reaction chamber 10 and the feeding chamber 20 are separated by a horizontal semi-permeable membrane 30. On the right side, the system comprises a reaction chamber 10 holding a reaction solution 12 and a feeding chamber 20 holding H2O 23. The reaction chamber 10 and the feeding chamber 20 are separated by a horizontal semi-permeable membrane 30.

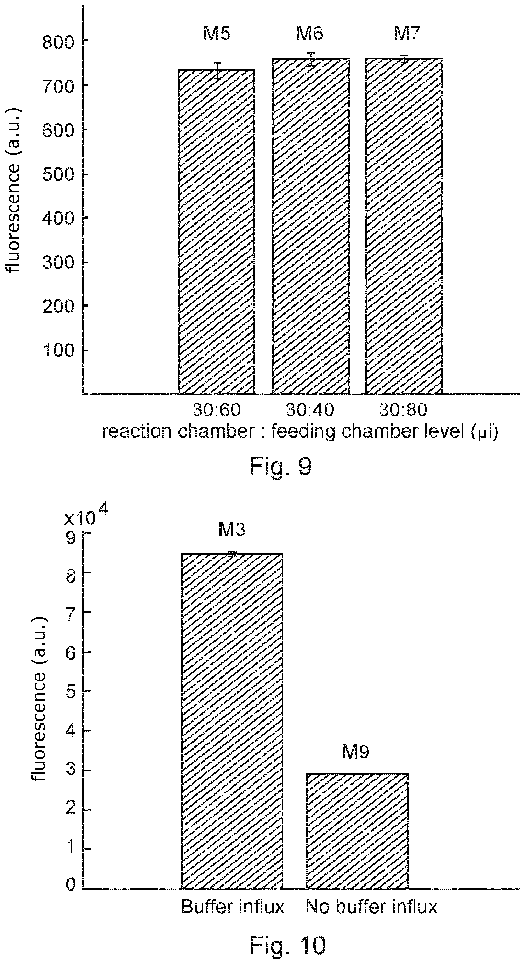

Fig. 9 shows a diagram comparing different relative levels of buffer solution and reaction solution. The diagram indicates an endlevel fluorescence measurement of a TX reaction in the system with continuous buffer influx with different relative levels of buffer and reaction solution in the chambers. A fifth measurement M5 indicates a relation of filling of the reaction chamber to the feeding chamber of 30ul:60ul leading to a fluorescence of 700 a.u. A sixth measurement M6 indicates a relation of filling of the reaction chamber to the feeding chamber of 30ul:40ul leading to a fluorescence of 750 a.u. A sixth measurement M6 indicates a relation of filling of the reaction chamber to the feeding chamber of 30ul:80ul leading to a fluorescence of 750 a.u. Consequently, the relation of filling of the reaction chamber to the feeding chamber seems to have only minor impact on the yield of the protein production.

Fig. 10 shows a diagram comparing protein production with and without feeding solution in a chamber system with vertical semi-permeable membrane. The diagram indicates an endlevel fluorescence measurement of a vertical TX-TL reaction with mTurq as a fluorescent protein. An eighth measurement M8 indicates a protein production with a vertical semi-permeable membrane with a provided feeding solution leading to a fluorescence of 85000 a.u. A ninth measurement M9 indicates a protein production with a vertical semi-permeable membrane without a provided feeding solution leading to a fluorescence of 30000 a.u. Consequently, the feeding solution heavily improves the yield of the protein production.

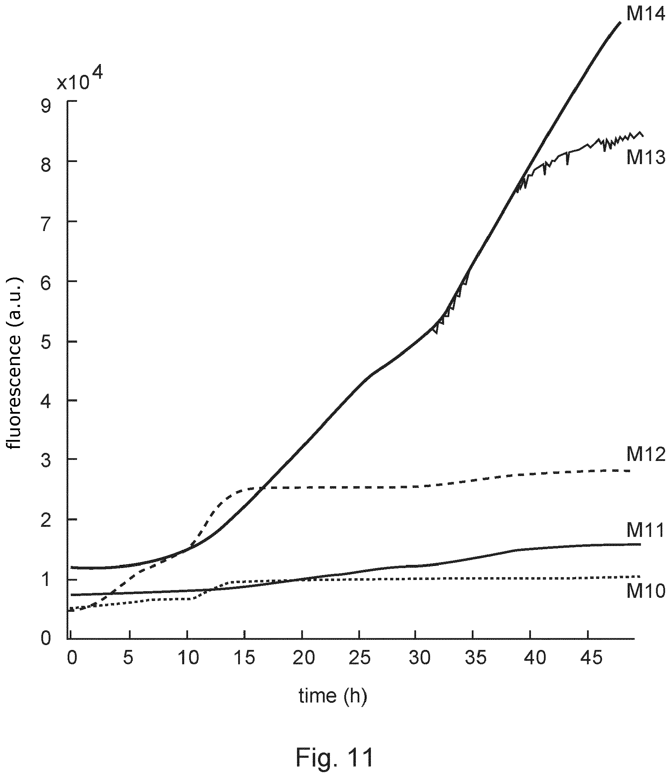

Fig. 11 shows a diagram comparing protein production with different conditions.

The diagram indicates comparison of TX-TL reaction with different conditions by fluorescence measurement for 50 h with mTurq as fluorescent protein. The cell extract reaction with a plasmid encoding a fluorescent protein is placed in the middle chamber while, in the two reservoir chambers outside, the supply buffer, which does not have the same composition as the cell extract reaction, is given. Subsequently, the typical kinetics of protein synthesis in a TX-TL reaction can be seen, which is divided into three phases. In the first phase, delays occur due to the maturation of the protein, which is why only a minimal increase in fluorescence is observed in the curve. The middle phase, an exponential increase in protein expression is observed and lasts approximately 1-6 hours. In the third phase, protein expression reaches a plateau, which is due to the depletion of biochemical building blocks (amino acids, ribonucleotides) and a change in biochemical conditions. In Figure 3, an expression curve in a TX-TL reaction has been compared with an expression curve in a TX-TL reaction with constant buffer feeding. In the expression without dialysis membrane, a classical expression curve was observed as described above. The stationary phase started after 30 minutes and ended after 12 hours of total reaction time. Thereafter, a constant plateau was reached. The expression curve of gene expression with constant buffer supply also showed an initial phase with a duration of 6 h. The steady state was observed after 6 hours. The steady state is reached only after 35-40 h reaction time.

A tenth measurement M10 indicates a chamber system with horizontal semi-permeable membrane without a provided feeding solution leading to an endlevel fluorescence of 8000 a.u. An eleventh measurement M11 indicates a chamber system with horizontal semi-permeable membrane with a provided feeding solution leading to an endlevel fluorescence of 12000 a.u. A twelfth measurement M12 indicates a chamber system with vertical semi-permeable membrane without a provided feeding solution leading to an endlevel fluorescence of 30000 a.u. A thirteenth measurement M13 indicates a chamber system with vertical semi-permeable membrane with a provided feeding solution leading to an endlevel fluorescence of 85000 a.u. A fourteenth measurement M14 indicates a chamber system with vertical semi-permeable membrane with a provided feeding solution that is constantly replenished via a feeding chamber access leading to an endlevel fluorescence of 100000 a.u. as compared to the thirteenth measurement M13 the steady state is not yet reached after 50h.

Example 1 provides a chamber system for cell-free production of proteins of interest, comprising at least one reaction chamber, being configured to hold a reaction solution and being configured to produce proteins of interest using the reaction solution, at least one feeding chamber, being disposed adjacent to the at least one reaction chamber and being configured to hold a feeding solution, and at least one semi-permeable membrane, connecting the at least one feeding chamber with the at least one reaction chamber, wherein the semi-permeable membrane is configured to allow diffusing at least part of the feeding solution from the at least one feeding chamber into the at least one reaction chamber.

Example 2 includes the subject-matter of example 1. In this example, the at least one feeding chamber comprises a feeding chamber access that is configured to allow access to the feeding solution in the at least one feeding chamber.

Example 3 includes the subject-matter of examples 1-2. In this example, the feeding chamber access is configured to allow access to the feedings solution during production of the proteins of interest.

Example 4 includes the subject-matter of examples 1-3. In this example, the feeding chamber access is disposed on a top surface of the at least one feeding chamber.

Example 5 includes the subject-matter of examples 1-4. In this example, the at least one semi-permeable membrane is vertically oriented between the at least one reaction chamber and the at least one feeding chamber.

Example 6 includes the subject-matter of examples 1-5. In this example, the at least one semi-permeable membrane is fixed between the at least one reaction chamber and the at least one feeding chamber using an adhesive.

Example 7 includes the subject-matter of example 6. In this example, the adhesive comprises silicon compounds.

Example 8 includes the subject-matter of examples 1-7. This example provides at least one reaction comb, wherein the at least one reaction comb comprises the at least one reaction chamber and at least one feeding comb, wherein the at least one feeding comb comprises the at least one feeding chamber. The at least one reaction comb is disposed adjacent to the at least one feeding comb in a width direction. Between the at least one reaction comb and the at least one feeding comb the at least one semi-permeable membrane is disposed, connecting the at least one reaction chamber with the at least one feeding chamber.

Example 9 includes the subject-matter of example 8. In this example, the reaction comb comprises a plurality of reaction chambers that are disposed adjacent to each other along a length direction perpendicular to the width direction Example 10 includes the subject-matter of examples 8-9. In this example, the feeding comb comprises a plurality of feeding chambers that are disposed adjacent to each other along the length direction perpendicular to the width direction.

Example 11 includes the subject-matter of examples 8-10. In this example, between the at least one reaction comb and the at least one feeding comb only one semi-permeable membrane is disposed, connecting the at least one reaction chamber with the at least one feeding chamber.

Example 12 includes the subject-matter of example 11. This example provides a first feeding comb and a second feeding comb. The first feeding comb and the second feeding comb are disposed adjacent to the at least one reaction comb at opposing sides of the at least one reaction comb in the width direction.

Example 13 includes the subject-matter of example 11. In this example, the first feeding comb and the second feeding comb comprise fastening means that extend towards each other in the width direction. The at least one reaction comb comprises at least one fastening opening through which the fastening means of the first feeding comb and the second feeding comb extend. The fastening means are configured to fasten the first feeding comb and the second feeding comb together, holding the reaction comb between the first feeding comb and the second feeding comb.

Example 14 includes the subject-matter of examples 1-13. This example provides a box, wherein the box comprises a bottom part, configured to receive the at least one reaction chamber and the at least one feeding chamber, and a cover part, configured to cover the bottom part.

Example 15 includes the subject-matter of examples 1-14. In this example, the semi-permeable membrane has a pore size of less than or equal to 2 nm.

Example 16 provides a method for cell-free production of proteins of interest using a chamber system of examples 1-14, comprising the steps. Providing the reaction solution to the at least one reaction chamber and the feeding solution to the at least one feeding chamber. Incubating the reaction solution, thereby producing the proteins of interest.

### List of reference symbols

- 10: reaction chamber
- 11: reaction chamber access
- 12: reaction solution
- 20: feeding chamber
- 21: feeding chamber access
- 22: feeding solution
- 23: H2O
- 30: semi-permeable membrane
- 40: fastening means
- 41: fastening protrusion
- 42: fastening recess
- 50: fastening opening
- 60: box
- 61: bottom part
- 62: top part
- 63: comb recess
- F: feeding comb
- F1: first feeding comb
- F2: second feeding comb
- R: reaction comb
- L: length direction
- W: width direction
- 100: chamber system
- 200: chamber system
- 300: chamber system
- M1-M14: measurements

## Claims

1. Chamber system for cell-free production of proteins of interest, comprising:
at least one reaction chamber (10), being configured to hold a reaction solution (12) and being configured to produce proteins of interest using the reaction solution (12);
at least one feeding chamber (20), being disposed adjacent to the at least one reaction chamber (10) and being configured to hold a feeding solution (22);
at least one semi-permeable membrane (30), connecting the at least one feeding chamber (20) with the at least one reaction chamber (10), wherein the semi-permeable membrane (30) is configured to allow diffusing at least part of the feeding solution (22) from the at least one feeding chamber (20) into the at least one reaction chamber (10);
wherein the at least one feeding chamber (20) comprises a feeding chamber access (21) that is configured to allow access to the feeding solution (22) in the at least one feeding chamber (20).

2. Chamber system of claim 1,
wherein the feeding chamber access (21) is disposed on a top surface of the at least one feeding chamber.

3. Chamber system of any one of the preceding claims,
wherein the feeding chamber access (21) is configured to allow access to the feedings solution (22) during production of the proteins of interest.

4. Chamber system of any one of the preceding claims,
wherein the at least one semi-permeable membrane (30) is vertically oriented between the at least one reaction chamber (10) and the at least one feeding chamber (20).

5. Chamber system of any one of the preceding claims,
wherein the at least one semi-permeable membrane (30) is fixed between the at least one reaction chamber (10) and the at least one feeding chamber (20) using an adhesive;
wherein preferably the adhesive comprises silicon compounds.

6. Chamber system of any one of the preceding claims,
wherein the protein of interest is a bacteriophage protein.

7. Chamber system of any one of the preceding claims, comprising:
at least one reaction comb (R), wherein the at least one reaction comb (R) comprises the at least one reaction chamber (10); and
at least one feeding comb (F), wherein the at least one feeding comb comprises the at least one feeding chamber (20);
wherein the at least one reaction comb (R) is disposed adjacent to the at least one feeding comb (F) in a width direction (W);
wherein between the at least one reaction comb (R) and the at least one feeding comb (F) the at least one semi-permeable membrane (30) is disposed, connecting the at least one reaction chamber (10) with the at least one feeding chamber (20).

8. Chamber system of claim 7,
wherein the reaction comb (R) comprises a plurality of reaction chambers (10) that are disposed adjacent to each other along a length direction (L) perpendicular to the width direction (W).

9. Chamber system of any one of claims 7 or 8,
wherein the feeding comb (F) comprises a plurality of feeding chambers (20) that are disposed adjacent to each other along the length direction (L) perpendicular to the width direction (W).

10. Chamber system of any one of claims 7 to 9,
wherein between the at least one reaction comb (R) and the at least one feeding comb (F) only one semi-permeable membrane (30) is disposed, connecting the at least one reaction chamber (10) with the at least one feeding chamber (20).

11. Chamber system of claim 10, comprising:
a first feeding comb (F 1) and a second feeding comb (F2);
wherein the first feeding comb (F1) and the second feeding comb (F2) are disposed adjacent to the at least one reaction comb (R) at opposing sides of the at least one reaction comb (R) in the width direction (W).

12. Chamber system of claim 11,
wherein the first feeding comb (F1) and the second feeding comb (F2) comprise fastening means (40) that extend towards each other in the width direction (W);
wherein the at least one reaction comb (R) comprises at least one fastening opening (50) through which the fastening means (40) of the first feeding comb (F1) and the second feeding comb (F2) extend;
wherein the fastening means (40) are configured to fasten the first feeding comb (F1) and the second feeding comb (F2) together, holding the reaction comb (R) between the first feeding comb (F1) and the second feeding comb (F2).

13. Chamber system of any one of the preceding claims, comprising
a box (60), wherein the box comprises a bottom part (61), configured to receive the at least one reaction chamber (10) and the at least one feeding chamber (20), and a cover part (62), configured to cover the bottom part (61).

14. Chamber system of any one of the preceding claims,
wherein the semi-permeable membrane (30) has a pore size of less than or equal to 2 nm.

15. Method for cell-free production of proteins of interest using a chamber system of any of the claims 1-14, comprising the steps:
providing the reaction solution (12) to the at least one reaction chamber (10) and the feeding solution (22) to the at least one feeding chamber (20);
incubating the reaction solution (12), thereby producing the proteins of interest.
